# EUROPEAN PATENT APPLICATION

(11) **EP 1 661 585 A1**
(43) Date of publication of application: **31.05.2006**
(21) Application number: 05257161.9
(22) Date of filing: 21.11.2005
(51) Int. Cl.: A61L 2/00, A61L 2/16

(54) **Method of preventing the development of biocide resistant bacteria**

(30) Priority: 22.11.2004 GB 0425656
(71) Applicant: Cleenol Group Ltd, Banbury Oxfordshire OX16 1RB (GB)
(72) Inventor: Greaves, Richard Stephen, Norton Oxfordshire OX7 3NY (GB)
(74) Representative: Baldock, Sharon Claire

(57) **Abstract**

A method for preventing the development of biocide resistant populations of bacteria, comprising the alternating use of at least first and second biocides in temporally separated manner, wherein the first biocide is used for a specified cleaning operation for a first designated period, and after said designated period the first biocide is replaced by a second biocide which is used for said cleaning operation for a second designated period.

## Description

The present invention relates to a method of preventing or reducing the incidence of the development of biocide resistant bacteria in a localised environment.

Bacteria are found in large numbers in the environment, this can result in contamination and associated problems in areas where large build ups of bacteria occur, or where food is prepared or stored, for example hotels, supermarkets or restaurants. The build up of bacteria can also lead to serious health issues in areas where immunocompromised individuals are present such as hospitals or nursing homes. It is therefore important to ensure that the environment in such areas is kept clean and relatively bacteria free.

Of particular interest and concern has been the development of biocide resistant strains of the widespread bacteria *Staphyloccus Aureus.* This Bacteria is commonly found in the environment and only becomes of concern medically when it infects wounds. In general *S. Aureus* infection has been treated with the antibiotic methycillin. However, the recent development of strains of methycillin resistant *S*. *Aureus* (MRSA) has meant that it has become even more critical that locations where such bacterial infections could occur are kept clean and bacteria free.

The development of such antibiotic resistant bacterial strains occurs when normal bacteria develop resistance to biocides used in the environment, resulting in the development of mutated bacterial populations, these mutant populations may also be resistant to antibiotics and can result in serious medical problems, even death, should they enter the body.

It is an object of the present invention to overcome the problem of the development of biocide resistant populations of bacteria through the use of a novel biocide regimen.

According to a first aspect of the present invention there is provided a method of preventing the development of biocide resistant populations of bacteria in an environment, said method comprising the alternating use of at least first and second biocides in a temporally separated manner, wherein, the first biocide is used for a specified cleaning operation for a first designated period, after said designated period, the first biocide is replaced by the second biocide which is used for said cleaning operation for a second designated period.

It will be understood that after the second designated period, the second biocide will be again replaced either by the first biocide, or a further biocide for a further designated period, and that this cyclical use will be continued. Furthermore, the first and second designated periods may be of the same or different. Preferably, the designated periods are at least 24 hours, more preferably between at least 7 days and 1 calendar month.

Preferably, the first and second biocides have different modes of action in killing bacteria. Preferably, if further biocides are used, these will again have a different mode of action to the first and/or second biocide.

It will be further understood that the term cleaning operation refers to the cleaning of a particular environment or area and is not limited to a specified mode of cleaning, for example, wiping a surface with a biocide impregnated cloth or spraying the same surface with a biocidal aerosol maybe considered the same cleaning operation.

Preferably, the first biocide is a quaternary ammonium compound (QAC).

Preferably, the second biocide is biguanide.

It will be understood that the term biocide as used in the specification refers to any composition formulated to kill bacteria externally to the body, and that this term may be used interchangeably with the terms, bactericide, disinfectant and anti-microbial agent.

Preferably, the at least first and second biocides are colour coded to enable identification. More preferably, each biocide contains a colourant, such as, for example, a dye or pigment. Even more preferably, the colourants do not discolour the environment or area in which they are to be used.

In a preferred embodiment of the present invention, the first and second biocides comprise different coloured concentrated solutions or liquids provided in separate containers. More preferably, the containers have specific identifying means thereon. Preferably the containers have at least a transparent region so that the colour of the biocide contained therein is visible. Preferably, the biocide containers are also differently colour coded so as to indicate their contents. It will however, be understood that other identification means, such as for example numerical, alphabetical or symbolic coding, may be employed to identify the biocide contained in the container.

The containers may be of any appropriate form. In a preferred embodiment the containers are pouches which can be fitted into a dispenser so as to dispense a volume of biocide which is then diluted appropriately for use.

It will be apparent to the skilled person that the biocides can be supplied in forms other than as a concentrated solution or liquid, which may be used neat or diluted by the user. They may, for example, be provided in solid form to be dissolved, or as pre-diluted solutions to be used directly, dependent upon the cleaning operation to be undertaken. Alternatively, the biocides may be supplied impregnated into pads or wipes to be used on surfaces forming the environment to be cleaned, or as a cream, aerosol or trigger spray.

The biocides maybe in admixture with other suitable cleaning products such as detergents or diluents to form a biocide containing composition suitable for the cleaning operations to be performed. It will be further understood that the specific environment in which the method is intended to be employed will determine the preferred form of the biocide containing composition chosen. For example, if the environment is an environment such as a hospital ward or an industrial environment then solutions which can be used to directly clean large surfaces may be preferable. However, if the environment is, for example, a commercial kitchen then a spray which can be used directly on food preparation surfaces may be preferred.

According to a second aspect of the current invention there is provided a method of supplying at least first and second biocides for use in the method according to the first aspect, said method comprising supplying the first and second biocides to a user in an alternating manner, wherein upon supply of the first biocide, a system is triggered such that when a further supply of biocide is required by the user the second biocide is supplied, so as to ensure that the biocides are used in the correct temporally separated manner.

It will be readily apparent that the supply operation may be repeated *ad infinitum* alternating between the at least first and second biocides. Alternatively, after a defined period, further biocides may be substituted for the first and/or second biocides, providing a further safeguard against the development of biocide resistant populations of bacteria.

Alternatively, the at least first and second biocides may be supplied simultaneously as part of a kit further comprising instructions as to which biocide is for first use, and the time period, or number of cleaning operations, for which the first biocide should be used before the second supplied biocide is substituted in the cleaning operation.

An embodiment of the invention will now be described by way of example only.

Quaternary ammonium compound (QAC), also known as quaternary ammonium halide or benzalkonium chloride, is a colourless, odourless biocide commonly used as a disinfectant. This is supplied to the end user as a concentrated solution containing a dye which is added at a low concentration so as not to discolour the area which is being cleaned. The solution is diluted by the user to an appropriate concentration for use in the cleaning of specific areas, for example, hospital wards or commercial kitchens. The QAC is supplied in a container in the form of a pouch of an appropriate volume which can be fitted into a dispenser for dispensing the concentrated biocide. The pouch contains a window to allow the colour of the biocide to be seen, and also carries a means of identifying the contents of the container as QAC. When the supply of the QAC biocide is exhausted, or running low, the user is supplied with a further stock of a second concentrated cleaning solution containing biguanide rather than QAC. The second biguanide biocide contains a dye of a different colour to that present in the QAC. The biguanide is supplied in a pouch container of a suitable volume containing a window and having a means of identifying the contents of the same type as that present on the QAC pouch, which enables the biguanide container to be distinguished from that containing QAC.

The process is then repeated, alternating the biocide supplied between QAC and biguanide. This significantly reduces the risk of populations of biocide resistant bacteria developing because the bacteria are exposed to biocides having different modes of action. QAC and biguanide are effective against different bacteria at different levels as shown in table I, such that the cyclical nature of biocide use means that where one biocide is not effective against a particular form of bacteria, the other may have a much lower inhibitory concentration.

**TABLE I**

| Bacterial species | Biguanide conc. required to stop bacterial growth(ppm) | QAC conc. required to stop bacterial growth (ppm) |
|---|---|---|
| *Pseudomonas Aeriginosa* | 100 | 200 |
| *Bacillus Subtillus* | 10 | 30 |
| *Aureobasidium Pullulans* | 1250 | 25 |
| *Eschericia Coli* | 750 | 75 |

Records of the biocide previously supplied to a customer are retained, such that when the customer reorders the biocide containing solution, that containing the alternate biocide to the previously supplied solution is provided. For customers where a regular order of cleaning fluid is fulfilled, cleaning solutions containing QAC or biguanide are supplied in an alternating manner, under the control of a purchase/audit system.

In a further embodiment, the cleaning solutions containing the different biocides are supplied at the same time as part of a kit along with instructions for use. The cleaning solutions are supplied as colour coded solutions in first and second colour coded containers indicative of whether the biocide contained therein is QAC or biguanide. The contents of the first container are used for a cleaning operation for a specified time period, for example 1 week, 1 month, or until all the cleaning solution has been used, as described in the instructions contained in the kit. The second cleaning solution is then used for the cleaning operation for a second specified period which may be the same or different from the first.

## Claims

1. A method for preventing the development of biocide resistant populations of bacteria, said method comprising the alternating use of at least first and second biocides in a temporally separated manner, wherein, the first biocide is used for a specified cleaning operation for a first designated period, and after said designated period, the first biocide is replaced by a second biocide which is used for said cleaning operation for a second designated period.

2. A method according to claim 1 wherein the at least first and second biocides have different modes of action in killing bacteria.

3. A method according to claim 1 or 2, wherein the first biocide is a quaternary ammonium compound (QAC).

4. A method according to any one of claim 1 to 3, wherein the second biocide is biguanide.

5. A method according to any preceding claim, wherein the at least first and second biocides are colour coded to enable identification.

6. A method according to claim 5, wherein each biocide contains a colourant of a different colour.

7. A method according to any preceding claim, wherein the first and second biocides are provided in first and second containers having specific identifying means thereon.

8. A method according to claim 7, wherein the biocide containers are colour coded so as to indicate their contents.

9. A method of supplying at least first and second biocides for use in the method of any preceding claim, said method comprising supplying the first and second biocide to a user in an alternating manner, wherein upon supply of the first biocide, a system is triggered such that when further biocide is supplied, the second biocide is supplied to ensure that the biocides are used in the correct temporally separated manner.

10. A method according to claim 9, wherein the first and second biocides are provided in separate containers having specific identifying means thereon.

11. A method according to claim 10, wherein the biocide containers are colour coded so as to indicate their contents.

12. A kit comprising first and second biocides according to any one of claims 5 to 8 and instructions indicating that the first biocide should be used in a cleaning operation for a specified time period or number of cleaning operations before the second supplied biocide is substituted for use in the cleaning operation.
